(19) Europäisches Patentamt
European Patent Office
Office européen des brevets

(11) Veröffentlichungsnummer: **0 273 199**
**A2**

# (12) EUROPÄISCHE PATENTANMELDUNG

(21) Anmeldenummer: **87117379.5**

(22) Anmeldetag: **25.11.87**

(51) Int. Cl.⁴: **C07D 211/08**, C07D 211/22, A61K 31/445

Patentansprüche für folgenden Vertragsstaaten: GR + ES.

(30) Priorität: **27.11.86 DE 3640475**

(43) Veröffentlichungstag der Anmeldung: **06.07.88 Patentblatt 88/27**

(84) Benannte Vertragsstaaten: **AT BE CH DE ES FR GB GR IT LI LU NL SE**

(71) Anmelder: **LABORATOIRES HOECHST S.A.**
**1 Terrasse Bellini**
**F-92800 Puteaux(FR)**

(72) Erfinder: **Petit, Serge**
**113, rue Francis de Pressensé**
**F-69100 Villeurbanne(FR)**
Erfinder: **Nallet, Jean-Pierre**
**400, rue du Bacon Montenay**
**F-69300 Neuville s/Saone(FR)**
Erfinder: **Dreux, Jacques**
**36, rue Raulin**
**F-69007 Lyon(FR)**
Erfinder: **Winicki, Jacqueline**
**34, rue Saint-James**
**F-92200 Neuilly(FR)**

(74) Vertreter: **Meyer-Dulheuer, Karl-Hermann, Dr. et al**
**HOECHST Aktiengesellschaft Zentrale Patentabteilung Postfach 80 03 20**
**D-6230 Frankfurt/Main 80(DE)**

## (54) Arzneimittel auf Basis von Derivaten des 3,4-Diphenylpiperidins, die Verwendung der Derivate als Arzneimittel sowie neue 3,4-Diphenylpiperidinderivate und Verfahren zu ihrer Herstellung.

(57) Arzneimittel enthaltend ein 3,4-Diphenylpiperidinderivat der Formel Ia

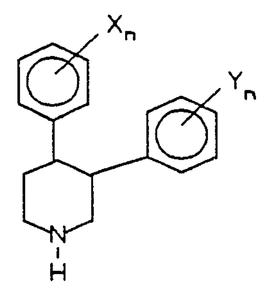

Ia

worin X, Y und n die angegebenen Bedeutungen haben, oder ein physiologisch verträgliches Salz dieser Verbindungen sowei deren Verwendung als Antidepressivum werden beschrieben. Außerdem werden 3,4-Diphenylpiperidinderivate der Formel I

EP 0 273 199 A2

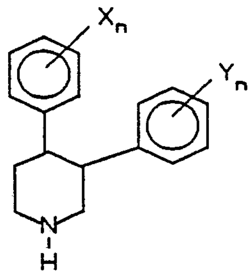

I

worin X, Y und n die angegebenen Bedeutungen haben und deren physiologisch verträgliche Salze sowie Verfahren zu ihrer Herstellung beschrieben.

**Arzneimittel auf Basis von Derivaten des 3,4-Diphenylpiperidins, die Verwendung der Derivate als Arzneimittel sowie neue 3,4-Diphenylpiperidinderivate und Verfahren zu ihrer Herstellung**

Die vorliegende Erfindung betrifft Arzneimittel enthaltend 3,4-Diphenylpiperidin, ein 3,4-Diphenylpiperidinderivat oder ein physiologisch verträgliches Salz dieser Verbindungen, die Verwendung dieser Verbindungen als Arzneimittel und dessen Herstellung. Die Erfindung betrifft ferner neue, in einem oder in beiden Phenylresten substituierte 3,4-Diphenylpiperidinderivate und deren physiologisch verträgliche Salze sowie Verfahren zur Herstellung dieser Verbindungen.

In der Literatur finden sich einige Hinweise auf die Klasse der 3,4-Diphenylpiperidine.

So beschreibt z.B. C.F. Koelsch, J. Am. Chem. Soc., 65, 2093-5, 1943, chemisch die beiden α-und β-Isomeren des 3,4-Diphenylpiperidins.

E.J. Fellows, Proc. Soc. Exptl. Biol. Med. 57, 276-7, 1944, beschreibt eine lokalanästhetische Wirkung der α-und β-Isomeren des 3,4-Diphenylpiperidins am Meerschweinchen und Kaninchen.

Ferner beschreibt V. Nacci, Farmaco. Ed. Sc., 328(5), 399 - 410, 1973, ein Chlorhydrat des 4-(3,4-Dimethoxyphenyl)-3-phenylpiperidins und dessen zytostatische und zytotoxische Eigenschaften beim Kontakt mit KB-Zellen in vitro.

Cis-4-(m-Aminophenyl)-3-phenylpiperidin und seine Herstellung wird von C.F. Koelsch et al. in J. Am. Chem. Soc. 66, 1957 (1944) beschrieben.

Bei Wiederaufnahme der Untersuchung von 3,4-Diphenylderivaten des Piperidins hat die Anmelderin nun am Tier pharmakologische Wirkungen, die auf eine antidepressive Wirkung am Menschen hinweisen, sowie in noch überraschender Weise gefunden, daß gewisse solche Moleküle in Dosierungen, wo sie in Tests für Antidepressiva eine starke Wirkung aufweisen, keine psychostimulierende Wirkung besitzen. Das Fehlen einer psychostimulierenden Wirkung war insofern überraschend, da von einigen 3,4-Diphenyl-2-piperidonen eine psychostimulierende Wirkung bei gleichzeitiger antidepressiver Wirkung beschrieben wurde.

Gegenstand vorliegender Erfindung sind Arzneimittel mit antidepressiver Wirkung und insbesondere antidepressiv wirkende Arzneimittel ohne psychostimulierende Wirkung, welche gegebenenfalls analgetische Wikrung besitzen, dadurch gekennzeichnet, daß sie ein cis-und/oder trans-3,4-Diphenylpiperidinderivat der Formel (Ia)

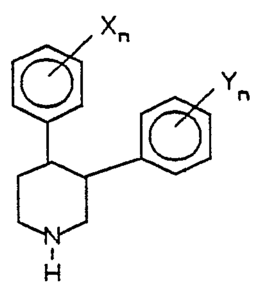

Ia

worin

X und Y gleich oder verschieden sind und für Wasserstoff, Halogen, $C_1$-$C_4$-Alkyl, $C_5$-$C_6$-Cycloalkyl, Phenyl-($C_1$-$C_3$)-alkyl, Trifluormethyl, Trichlormethyl, Hydroxy, $C_1$-$C_4$-Alkoxy, Halogen-($C_1$-$C_4$)-alkoxy, Phenyloxy, Phenyl-($C_1$-$C_3$)-alkoxy, Acyloxy mit bis zu 4 Kohlenstofffatomen, Amino, Nitro, N,N-Dialkylamino mit jeweils 1 bis 4 Kohlenstoffatomen in den Alkylresten, Morpholino oder $C_1$-$C_4$-Alkylsulfonyl stehen und

n eins, zwei oder drei bedeutet,

oder ein physiologisch verträgliches Salz dieser Verbindungen enthalten.

Die Verbindungen können in Form von Racematen oder Enantiomeren vorliegen.

Falls n zwei oder drei bedeutet, so sind die Substituenten in dem jeweiligen Kern gleich oder verschieden.

Die Alkyl-und Alkoxygruppen können geradkettig oder verzweigt sein.

Die pharmazeutischen Zusammensetzungen können in geeigneter Form zur peroralen, rektalen oder parenteralen Verabreichung, beispielsweise in Form von Hartkapseln, Tabletten, Granulat, Weichkapseln oder wäßrigen Lösungen, Sirups oder trinkbaren Suspensionen, vorliegen. Die Zubereitungen enthalten den Wirkstoff entweder in freier Form oder als pharamkologisch verträgliches Salz und vorzugsweise pharmazeutisch unbedenkliche Zusatzstoffe, wie Verdünnungsmittel und/oder Trägermaterialien. Hierunter

sind physiologisch unbedenkliche Substanzen zu verstehen, die nach dem Vermischen mit dem Wirkstoff diesen in eine für die Verabreichung geeignete Form bringen.

Als häufig verwendete Trägerstoffe bzw. Verdünnungsmittel seien z.B. verschiedene Zucker oder Stärkearten, Cellulosederivate, Magnesiumcarbonat, Magnesiumstearat, Talk, Gelatine, kolloidales Siliciumdioxid, tierische und pflanzliche Öle, Polyethylenglykole, Wasser oder andere geeignete Lösungsmittel sowie wasserhaltige Puffermittel, die durch Zusatz von Glucose oder Salzen isotonisch gemacht werden können, genannt. Außerdem können gegebenfalls oberflächenaktive Mittel, Farb-und Geschmacksstoffe, Stabilisatoren, sowie Konservierungsmittel als weitere Zusatzstoffe in den erfindungsgemäßen Arzneimittelzubereitungen Verwendung finden. Der Wirkstoffanteil beträgt 10 bis 90 Gew.-%, vorzugsweise 25 bis 75 Gew.-%.

Die tägliche Dosierung des Wirkprinzips (d.h. des Moleküls in Form der Base oder pharmazeutisch unbedenklicher Salze) kann 5 bis 500 mg p.o. betragen. Die zu verabreichende Menge kann auf mehrere täglich zu verabreichende Dosierungseinheiten verteilt werden. Eine Dosierungseinheit, insbesondere für die orale Applikation, kann bis zu 500 mg, bevorzugt jeodch 5 bis 100 mg, des aktiven Bestandteils enthalten. Jedoch können auch darüber oder darunter liegende Dosierungseinheiten verwendet werden, die gegebenenfalls vor Verabreichung zu teilen bzw. zu vervielfachen sind. Gegebenenfalls können die Dosierungseinheiten für die orale Verabreichung mikroverkapselt werden, um die Abgabe zu Verzögern oder über einen längeren Zeitraum auszudehnen, wie beispielsweise durch Überziehen oder Einbetten von teilchenförmigem Material in geeignete Polymere, Wachse oder dergleichen.

Die parenterale Verabreichung kann unter Verwendung flüssiger Dosierungsformen, wie steriler Lösungen und Suspensionen erfolgen, die für die intramuskuläre, oder subkutane Injektion bestimmt sind. Derartige Dosierungsformen werden durch Lösen bzw. Suspendieren einer abgemessenen Wirkstoffmenge in einem geeigneten physiologisch unbedenklichen Verdünnungsmittel wie beispielsweise einem wäßrigen oder öligen Medium und Sterilisierung der Lösung bzw. der Suspension, gegebenenfalls unter Mitverwendung von geeigneten Stabilisatoren, Emulgatoren und/oder Konservierungsmitteln, hergestellt.

Die pharmazeutischen Präparate werden nach allgemein üblichen Verfahren hergestellt.

Verbindungen, die bei den Tests auf antidepressive Wirksamkeit am Menschen eine sehr hohe Aktivität und in derselben Dosis bei den Tests auf psychostimulierende Wirksamkeit eine sehr geringe oder keine Aktivität voraussagen, stellen die bevorzugten Wirkstoffe in den erfindungsgemäßen Zubereitungen dar:
trans-3,4-Diphenylpiperidin,
trans-4-(4-Methylphenyl)-3-phenylpiperidin,
trans-4-(4-Methoxyphenyl)-3-phenylpiperidin,
cis-4-(4-Chlorphenyl)-3-phenylpiperidin
trans-3-Phenyl-4-(4-trifluormethylphenyl)-piperidin
und deren pharmazeutisch unbedenkliche Salze.

Die vorliegende Erfindung betrifft ferner neue, substituierte 3,4-Diphenylpiperidinderivate der Formel I

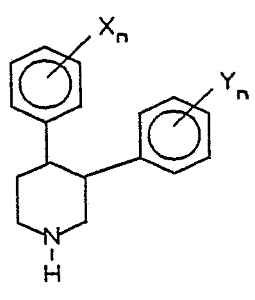

I

worin
X und Y gleich oder verschieden sind und für Halogen, $C_1$-$C_4$-Alkyl, $C_5$-$C_6$-Cycloalkyl, Phenyl-($C_1$-$C_3$)alkyl, Trifluormethyl, Trichlormethyl, Hydroxy, $C_1$-$C_4$-Alkoxy, Halogen-($C_1$-$C_4$)-alkoxy, Phenyloxy, Phenyl($C_1$-$C_3$)-alkoxy, Acyloxy mit bis zu 4 Kohlenstofffatomen, Nitro, N,N-Dialkylamino mit jeweils 1 bis 4 Kohlenstoffatomen in den Alkylresten, Morpholino oder $C_1$-$C_4$-Alkylsulfonyl stehen oder einer der Reste X oder Y Wasserstoff darstellt, und
n eins, zwei oder drei bedeutet,
oder ein physiologisch verträgliches Salz dieser Verbindungen, ausgenommen Verbindungen, worin einer der Substituenten X oder Y $C_1$-$C_4$-Alkoxy und der andere Wasserstoff und n gleich 2 ist.

Die Verbindungen liegen in der cis-oder trans-Form vor. Die Formel I umfaßt die Racemate und die Enantiomeren.

Falls n zwei oder drei bedeutet, so sind die Substituenten in dem jeweiligen Kern gleich oder verschieden.

Die Alkyl-und Alkoxygruppen sind geradkettig oder verzweigt.

Die Erfindung betrifft auch ein Verfahren zur Herstellung von Verbindungen der Formel I, welches dadurch gekennzeichnet ist, daß man 3,4-Diphenylpiperidone der Formel II

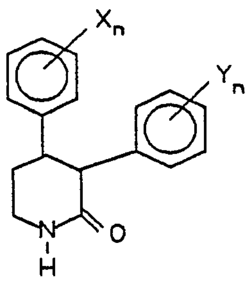

II

worin X, Y und n die zu Formel I angegebenen Bedeutungen haben, reduziert und gegebenenfalls in die physiologisch verträglichen Salze überführt.

Die Verbindungen der Formel II werden mit Hilfe eines Reduktionsmittels reduziert. Dabei bedetuet Reduktionsmittel jegliches, unter entsprechenden Bedingungen eingesetztes Reagenz, das ohne Reduktion der aromatischen Kerne die Amidfunktion zur Aminfunktion reduziert und gegebenenfalls dabei keine Isomerisierung der cis-Stereochemie zur tranas-Stereochemie verursacht.

So gestattet es das in Äthyläther bei einer Temperatur zwischen -10°C und der Rückflußtemperatur eingesetzte Doppelhydrid des Lithiums und Aluminiums ($LiAlH_4$), cis-Piperidine aus cis-Piperidonen zu erhalten.

Zur Reduktion von trans-Piperidonen zu trans-Piperidinen kann man $LiAlH_4$ in Dioxan bei Rückflußtemperatur verwenden. Eine Lösung von Diboran in THF ermöglicht die Reduktion von cis-oder trans-Piperidonen zu Piperidine derselben Stereochemie bei einer Temperatur zwischen 0°C und der Rückflußtemperatur.

Die Reste X und Y können auch nachträglich in 3,4-Diphenylpiperidinderivate eingeführt werden.

Verbindungen der Formel II erhält man z.B. aus den entsprechenden 2,3-Diphenyl-$\gamma$-nitrilesterderivaten (vgl. Europäische Patentanmeldung mit der Veröffentlichungs-Nr. 0 203 308 entspr. US-Patentanmeldung Nr. 847.255).

Die nachfolgende ergänzende Beschreibung, die einen Bericht bezüglich der Versuche zur pharmakologischen Wirksamkeit der Produkte der Formel I bzw. Ia sowie Herstellungsbeispiele für die erfindungsgemäßen Verbindungen der Formel I betrifft, soll dem besseren Verständnis der Erfindung dienen.

Es versteht sich jedoch, daß dieser Bericht und diese Beispiele ausschließlich der Erläuterung des Erfindungsgegenstands dienen und in keiner Weise eine Einschränkung darstellen.

Die pharmakologischen Test wurden an männlichen Ratten von 170 bis 220 g Gewicht (Wistar-Stamm) und männlichen Mäusen von 18 bis 23 g Gewicht (NMRI-Stamm) durchgeführt.

Sämtliche Versuche wurden als Blindversuche durchgeführt (d.h. bei der Durchführung der Tests ist dem Forscher nicht bekannt, welche Tiere die betreffende Wirksubstanz in welcher Dosis erhalten haben).

Falls nicht anders angegeben, wurden 10 Tiere pro Gruppe eingesetzt.

Sämtliche Tests wurden bei konstantgehaltener Laboratoriumstemperatur durchgeführt (22 + 1°C).

Die Testsubstanzen wurden in einer Suspension von 1 g Gummi arabicum pro 20 ml Wasser suspendiert.

Die Injektionsvolumina betrugen 0,5 ml pro 100 g Körpergewicht an der Ratte sowie 0,25 ml pro 20 g Körpergewicht bei der Maus.

Die akute Toxizität wurde intraperitoneal an der Maus für Gruppen von je drei Tieren bestimmt. Für jede Dosis wurde die Anzahl überlebender Tiere entweder sofort oder nach 24 Stunden Verzögerung angegeben. Für sämtliche erfindungsgemäße Produkte liegt die letale Dosis nicht unter 64 mg/kg, und in den meisten Fällen über 256 mg/kg (die höchste, im verwendeten Bereich verabreichte Dosis).

Die antidepressive Aktivität der Verbindungen zeigt sich in der Antireserpin-, Antiapomorphin-und Antioxotremorinaktivität bei der Maus sowie in der Wirkung auf die Toxizitität des Yohimbins bei der Maus. In diesen Untersuchungen wurde die erste Dosis festgehalten, bei der statistische Sicherheit erreicht wurde. Für die erfindungsgemäßen Verbindungen insgesamt liegt die erste aktive Dosis zwischen 0,5 mg/kg und 16 mg/kg i.p. oder p.o.

Die Substanzen wurden einer Reihe von orientierenden Tests, die auf antidepressive Akivität beim Menschen hinweisen, unterzogen: Reserpintest (an der Maus) - Antagonismus der Apomorphin-und Oxotremorinwirkungen an der Maus - Zunahme der Toxizität des Yohimbins (an der Maus) - Tests auf verzweifeltes Verhalten und Kopfzuckungen (an der Maus). Falls die Substanzen nach i.p. Verabreichung eine interessante Wirkkung zeigten, wurden die Substanzen auch nach p.o. Verabreichung untersucht.

**Antireserpinaktivität**

Zur Zeit 0 erhalten die Mäuse intraperitoneal Reserpin (2,5 mg/kg).

4 Stunden danach, wenn die Hypothermie genügend stark erscheint (30 bis 33°C), verabreicht man die verschiedenen zu untersuchenden Verbindungen i.p. oder p.o. Alle 30 Minuten über einen Zeitraum von 2 Stunden mißt man die Rektaltemperatur mit einer auf konstante Tiefe eingeführten, ein Thermoelement tragenden Sonde. Die Rektaltemperatur der mit verschiedenen erfindungsgemäßen Verbindungen behandelten Tiere wird mit der Temperatur einer Kontrollgruppe verglichen, die destilliertes Wasser auf die gleiche Weise wie die untersuchten Verbindungen erhält.

Die Akinesie (fehlende Bewegungen) und Ptose (Öffnungsgrad des Augenlids von 0 bis 4 nach Rubin u.a 1957) werden ebenfalls alle 30 Minuten über einen Zeitraum von 2 Stunden bewertet.

Die eine antidepressive Aktivität besitzenden Produkte wirken der durch Reserpin induzierten Hypothermie entgegen. Für jede Dosis der verabreichten Verbindung berechnet man die prozentuale Hemmung der Hypothermie.

**Antiapomorphinaktivität**

Zur Zeit 0 nach Ausgleich der Reaktaltemperaturen werden die Mäuse (6 pro Gruppe) in deren Beobachtung gestattende Einzelkäfige (11 x 3,5 x 4 cm) eingeetzt. 30 Minuten nach i.p. bzw. p.o. Verabreichung der Testsubstanz erhalten sie Apomorphin subkutan in einer Dosis von 16 mg/kg. Die Rektaltemperatur wird 30 Minuten nach dem Apomorphin gemessen. Die Kontrolltiere erhalten destilliertes Wasser statt der zu untersuchenden Verbindungen. Die bei diesen Kontrolltieren, die 16 mg/kg Apomorphin erhalten haben, beobachtete Hypothermie liegt im Durchschnitt bei 33°C. Für jede Verbindungsdosis berechnet man die prozentuale Hemmung der Hypothermie.

**Antioxotremorinaktivität**

Nach Ausgleich der Rektaltemperaturen erhalten Mäuse (6 pro Gruppe) entweder destillietes Wasser oder die verschiedenen zu untersuchenden Verbindungen i.p.

30 Minuten danach verabreicht man intraperitoneal Oxotremorin in einer Dosis von 0,5 mg/kg.

Die Mäuse werden dan beobachtet: Tremor wird je nach der Intensität von 0 bis 3 bewertet, und die Akinesie wird alle 10 Minuten über einen Zeitraum von 30 Minuten auf Vorliegen oder Fehlen bestimmt.

Außerdem beobachtet man 2 Stunden lang das Erscheinen peripherer Symptome (Tränen, Darmentleerung). Die Rektaltemperatur wird alle 30 Minuten über eine Zeitraum von 2 Stunden nach der Verabreichung des Oxotremorins gemessen.

Die eine antidepressive Aktivität besitzenden Produkte wirken der durch Oxotremorin induzierten Hypothermie entgegen.

Für jede Dosis der Verbindung berechnet man die prozentuale Hemmung der Hypothermie.

**Wirkung auf die Toxizität des Yohimbins**

Zur Zeit 0 erhalten die Mäuse entweder destilliertes Wasser oder verschiedene zu untersuchende Verbindungen i.p. 30 Minuten danach verabreicht man ihnen subkutan eine infratoxische Dosis von 25 mg/kg Yohimbinchlorhydrat.

Die Mortalität wird jede Stunde über einen Zeitraum von 4 Stunden verfolgt und dann nach 24 Stunden notiert.

Die eine antidepressive Aktivität besitzenden Produkte verursachen eine Potenzierung der Toxizität des Yohimbins.

**Test auf verzweifeltes Verhalten,** wie von Porsolt u.a. (Arch. Int, Pharmacodyn. Ther. 229, 327 - 336, 1977) beschrieben.

Die Verbindungen werden intraperetoneal 30 Minuten vor dem Test verabreicht, und die Tiere werden vor dem erzwungenen Schwimmtest isoliert gehalten. Man bewertet die Dauer der Unbeweglichkeitsperioden in Sekunden ausgedrückt, die von der 2. bis zur 6. Minute nach dem Einsetzen des Tieres in Wasser gezählt werden.

**Schwanzaufhängungstest an der Maus** wie von Stéru u.a. beschrieben (Psychopharmacology 85, 367-370, 1985).

Die Substanzen werden intraperitoneal 30 Minuten vor dem Test verabreicht und die Tiese isoliert (10 Mäuse pro Dosis).

Man bewertet die Dauer der Unbeweglichkeit, ausgedrückt in Sekunden über die 6 Minuten des Tests. Man untersucht auf eine Verkürzung dieser Unbeweglichkeitszeit.

**Potenzierung der L-5-Hydroxytryptophanwirkungen** (L-5 HTP) wie von Ortmann u.a. beschrieben (Drug Development Research 4, 593-606, 1984)

L-5 HTP induziert an der Maus ruckartige Bewegungen des Kopfes (Kopfzuckungen). Die zu untersuchenden Substanzen werden den Tieren (10 Mäuse pro Dosis) intraperitoneal 30 Minuten vor dem L-5 HTP (4 mg/kg intraperitoneal) verabreicht. Die ruckartigen Kopfbewegungen und der Tremor werden 1 Minutre lang alle 10 Minuten im Verlauf einer Stunde bewertet. Desimipramin und Indalpin sind die Kontrol-(Vergleichs-)verbindungen. Ein antidepressiver Effekt zeigt sich durch eine Zunahme der ruckartigen Kopfbewegungen und des Tremors.

Die Substanzen wurden einigen orientierenden Tests auf psychostimulierende Aktivität unterzogen. Dabei suchte man nach Verbindungen, bei denen dieser Aktivitätstyp fehlt oder sich nur bei Dosierungen zeigt, die viel höher liegen als für die Ausübung einer Wirkung in den Orientierungstests auf antidepressive Aktivität.

Bei diesen Test handelt es sich um: Aktimetrie (an der Maus) - Suchen nach stereotypen Bewegungen (an der Ratte) -Wechselwirkung mit Barbituraten.

**Aktimetrie**

Die Motoraktivität wird mittels Photozellenaktimetern bestimmt (Boissier und Simon, Arch. Int. Pharmacodyn. 1965, 158, 212-221).

Die Mäuse (10 oder ein Vielfaches von 10 pro Gruppe) werden unmittelbar nach i.p. Verabreichung der zu untersuchenden Verbindungen in das Aktimeter eingesetzt. Die Motoraktivität wird danach bis zur 60. Minute gemessen. De Zähler werden nach 30 Minuten abgelesen und dann auf 0 zurückgestellt und im Verlauf von 30 Minuten alle 10 Minuten abgelesen.

Die Beweglichkeit der mit den verschiedenen Verbindungen behandelten Tiere wird mit derjenigen einer destilliertes Wasser erhaltenden Kontrollgruppe verglichen.

Die eine psychostimulierende Aktivität besitzenden Produkte verursachen eine Zunahme der Beweglichkeit.

**Suche nach stereotypen Bewegungen an der Ratte**

Unmittelbar nach i.p. oder p.o. Verabreichung der zu unteruchenden Verbindungen werden die Tiere (6 Ratten pro Gruppe) in 20 x 10 x 10 cm große, mit einem Edelstahldeckel mit Löchern von 1 cm Durchmesser versehene Einzelkäfige aus durchsichtigem Kunststoff eingesetzt. Die stereotypen Bewegungen (Bewegungen der Mundhöhle, Schnüffeln, Lecken oder Kauen) werden nach der von Simon und Chermat (J. Pharmacol. Paris, 1972, 3, 235-238) beschriebenen Methode alle 10 Minuten von 0 bis 3 bewertet, bis sie bei allen Tieren verschwunden sind.

**Antagonismus gegen durch Natriumbarbital induzierten Schlaf**

Die Tiere (6 Mäuse pro Gruppe) werden in 20 x 10 x 10 cm große Einzelkäfige aus durchsichtigem Kunststoff eingesetzt und erhalten i.p. oder p.o. die zu untersuchenden Verbindungen oder destilliertes Wasser 30 Minuten vor einer hypnotischen Dosis Natriumbarbital (180 mg/kg intaperitoneal).

Die Zeiten für die Unterdrückung und das Wiederauftreten des Aufrichtreflexes werden für jedes Tier notiert. Die Gegenwart einer psychlostimulierenden Aktivität zeigt sich in einer Gegenwirkung auf den durch Natriumbarbital induzierten Schlaf.

Gewisse Substanzen wurden einem analgetischen Test unterzogen.

**Heizplattentest:** Lecken und Springen, wie von Jacob und Blozovski beschrieben (Arch. Int. Pharmacodyn. 133, 296-209, 1961).

Die in Sekunden ausgedrückten Reaktionszeiten auf einer Heizplatte werden unter Berücksichtigung des Leckens der Vorderpfoten und Springen des Tieres als Kriterien bewertet. Die beiden Reaktionen werden am selben, anfänglich untrainierten Tier mit einer maximalen Ansprechzeit (Obergrenze) von 240 Sekunden beobachtet. Die Temperatur der Platte wird auf 56,6°C gehalten.

Morphin als positive Kontrolle wird subkutan und die übrigen Substanzen (mg/kg) werden intraperitoneal 30 Minuten vor dem Test verabreicht. Positives Ansprechen zeigt sich in einer Zunahme der Reaktionszeit.

Wird der schadensverhütenden Wirkung eine Substanz durch Naloxon entegengewirkt, so kann man - schließen, daß die Substanz tatsächlich eine analgetische Wirkung besitzt.

**ERGEBNISSE**

**Antidepressive Aktivität:**

Es wurde die erste Dosis angegeben, bei der statische Sicherheit erreicht wird. Für die Verbindungen der Formel I bzw. Ia insgesamt liegt diese erste aktive Dosis zwischen 0,5 mg/kg und 16 mg/kg intraperitoneal oder peroral.

Tabell 1 gibt Auskunft über antidepressive und psychostimulierende Aktivitäten einiger untersuchter Verbindungen der Formel I bzw. Ia. Falls für eine Verbindung mehrere Werte angegeben sind, so bezieht sich der niedrigste Wert auf i.p. Verabreichung.

Die in den Spalten 2 bis 5 zusammengestellten Werte geben Auskunft über die antidepressive Wirkung der Verbindungen, die Spalten 6 und 7 über die psychostimulierende Aktivität. Es ist erkennbar, daß nach Verabreichung der Testsubstanzen entweder die motorische Aktivität abnimmt (), nicht verändert wird (=) oder zunimmt (/). Der Test auf stereotype Bewegungen wurde bei beobachteter Abnahme der motorischen Aktivität nicht durchgeführt. Bei Zunahme der motorischen Aktivität sind in Spalte 6 die Dosen in mg/kg angegeben, die eine signifikante Erhöhung bewirken. Die Dosen, die eine Erhöhung der motorischen Aktivität bzw. eine stereotype Bewegung verursachen sind wesentlich höher als diejenigen, die eine positive Antwort im Test auf antidepressive Aktivität geben.

In Tabelle 1 b sind aktivitäten von Vergleichsverbindungen und der Verbindung gemäß Beispiel 3 zusammengestellt.

**Tabelle 1**

**Erste wirksame Dosis in mg/kg nach i.p. Verabreichung von Verbindungen der Formel I bzw. Ia in den 4 Haupttests auf antidepressive Wirkung**

| 1 | 2 | 3 | 4 | 5 | 6 | 7 |
|---|---|---|---|---|---|---|
| Verbindung gemäß Bsp. | Reserpin | Apomorphin 16 mg/kg | Oxotremorin | Yohimbin | Aktimetrie | Stereotype Bewegung |
| ** 2 | 4 | 1-2 | | | ↘ | |
| * 3 | 1 | 2-4 | 8 | 8 | ↘ | 0(64i.p.) |
| 7 (1.Stufe) | 32 | 0,5-1-4 | | 4-16 | ↘ (32i.p.) | |
| 10 (Base) | 16 | 16-32 | | 16 | ↘ | |
| 10 (HCl-Salz) | 4-16 | 16 | | | ↘ | |
| 11 | 4 | 1 | | 16 | = (32i.p.) | |
| 12 | 4 | 2 | 16 | 16 | ↘ = | 0(64i.p.) |
| 8 (Base) | 8 | 16 | | | ↘ | |
| 8 (HCl-Salz) | 4 | 8 | | 16 | ↘ | |
| 13 | 8 | 4 | | 16 | ↘ (32i.p.) | |
| 15 (HCl-Salz) | 1 | 1 | 0,25 | 1 | ↗ (8i.p.) | +(8 i.p.) |
| 15 (Base) | 0,5 | 0,5 | | 4 | ↗ (8i.p.) | |
| 18 | 0,5 | 0,5-2 | | 1 | ↗ (16i.p.) | +(16 p.o.) |
| 21 (Base) | 4 | 4 | 4 | 4 | = | 0(64 p.o.) |
| 21 (HCl-Salz) | 4 | 4 | | 16 | ↗ (32i.p.) | ±(64 p.o.) |

* tail suspension test= positiv ab 4 mg/kg i.p.
** " = positiv ab 8 mg/kg i.p.

Für sämtliche untersuchten Verbindungen der Formel I bzw. Ia wurde festgestellt, daß sie die motorische Aktivität (256 mg/kg verabreichte Maximaldosis) nicht erhöhen bzw. nur bei erheblich höheren Dosierungen als denen, die bei den Orientierungstests auf antidepressive Aktivität wirksam sind.

**Tabelle Ib**

Erste wirksame Dosis in mg/kg einiger bekannter Verbindungen im Vergleich zur Verbindung gemäß Beispiel 3

| Verbindung | Motilität | Apomorphin 1 $mg.kg^{-1}$ s.c. | Apomorphin 16 $mg.kg^{-1}$ s.c. | Reserpin | Oxotremorin | T.S.T. Maus | Yohimbin |
|---|---|---|---|---|---|---|---|
| IMIPRAMIN | ↘ 128 | 8 | 4 | 2 | 8 | 1-4 | 16 |
| DESIPRAMIN | ↘ 4 | > 16 | 2 | 0,25 | 0,125 | 1-4 | 0,5 |
| MIANSERIN | ↘ 32 | 64 | 64 | > 32 | 32 | 8 | 8 |
| NOMIFENSIN | ↗ 4 | 2 | 2 | 8 | 16 | 0,25 | 4 |
| VILOXAZIN | ↘ 16 | > 32 | 8 | 4 | 4 | 8 | 8 |
| Beispiel 3 | ↘ 32 | 2 | 2 | 1 | 8 | 4 | 8 |

Die Verbindungen wurden i.p. verabreicht.

Die in den Tests auf verzweifeltes Verhalten und Kopfzuckungen mit trans-3,4-Diphenylpiperidin (DPPT) und im Test auf Analgesie erhaltenen Ergebnisse sind im einzelnen angegeben.

**Test auf verzweifeltes Verhalten nach Porsolt:**

DPPT ab 8 mg/kg zeigt eine bedeutsame Abnahme in der Dauer der Unbeweglichkeit, wobei die Wirkung bei 16 mg/kg stärker ist. Bei dieser letzteren Dosis übt DPPT keinen stimulierenden Effekt an der Maus aus.

Die Wirkungen von Nomifensin, Desipramin und Imipramin in ungefähr gleichwertiger Dosis sind zum Vergleich in Tabelle 2 mit angegeben.

**Tabelle 2 "Vertweifeltes Verhalten" der Maus**

| Behandlung | Dosis [mg/kg] | n | Dauer der Unbeweglichkeit | % Antagonismus |
|---|---|---|---|---|
| Kontrolle | | 10 | 194 ± 16 | |
| DPPT | 8 | 10 | 141 ± 16 $p < 0,02$ | 28 % |
| Kontrolle | | 10 | 178 ± 11 | |
| DPPT | 16 | 10 | 94 ± 17 $p < 0,001$ | 47 % |
| Kontrolle | | 20 | 197 ± 9 | |
| Nomifensin | 4 | 10 | 120 ± 23 $p < 0,001$ | 39 % |
| Kontrolle | | 20 | 201 ± 9 | |
| Desipramin | 8 | 20 | 105 ± 16 $p < 0,001$ | 48 % |
| Kontrolle | | 10 | 178 ± 11 | |
| Imipramin | 16 | 10 | 112 ± 23 $p < 0,05$ | 37 % |

**"Tail suspension"-Test** (Schwanzaufhängungstest an der Maus) :

DPPT zeigt ab 2 mg/kg eine bedeutsame Verkürzung der Dauer der Unbeweglichkeit. Zum Vergleich sind die mit Imipramin und Nomifensin erhaltenen Ergebnisse in Tabelle 3 mit angegeben.

**Tabelle 3 Schwanzaufhängungstest an der Maus**

| Behandlung | Dosis [mg/kg] | Dauer der Unbeweglichkeit | % Antagonismus |
|---|---|---|---|
| Kontrolle | | 77 ± 9 | |
| DPPT | 1 | 58 ± 13 | 25 % |
| | 2 | 38 ± 7 $p < 0{,}01$ | 51 % |
| | 4 | 16 ± 2 $p < 0{,}001$ | 79 % |
| | 16 | 12 ± 3 $p < 0{,}001$ | 84 % |
| Kontrolle | | 102 ± 8 | |
| Imipramin | 1 | 70 ± 17 | 31 % |
| | 2 | 59 ± 8 $p < 0{,}01$ | 42 % |
| | 4 | 53 ± 11 $p < 0{,}01$ | 48 % |
| | 8 | 48 ± 10 $p < 0{,}01$ | 58 % |
| | 16 | 28 ± 7 $p < 0{,}001$ | 73 % |
| Kontrolle | | 91 ± 6 | |
| Nomifensin | 0,06 | 59 ± 9 | 35 % |
| | 0,25 | 41 ± 6 $p < 0{,}01$ | 55 % |
| | 1 | 31 ± 6 $p < 0{,}01$ | 66 % |
| | 4 | 18 ± 5 $p < 0{,}001$ | 80 % |

**Potenzierung von L-5 HTP**

DPPT führt zu einer Zunahme der durch L-5 HTP induzierten ruckartigen Kopfbewegungen (Kopfzuckungen) an der Maus. Der Effekt ist deutlich und bei 8 mg/kg gesichert. Der Tremor ist ebenfalls verstärkt (Tabelle 4).

**Tabelle 4 Potenzierung von L-5-Hydroxytryptophan an der Maus**

| Behandlung | Dosis [mg/kg] | ruckartige Kopfbewegungen | Tremor |
|---|---|---|---|
| Kontrolle | | 2,3 ± 0,3 | 0 |
| DPPT | 8 | 4,7 ± 0,6 $p < 0,01$ | 1,3 ± 0,4 |
| Kontrolle | | 2,1 ± 0,3 | 0 |
| Desipramin | 4 | 3,8 ± 0,4 $p < 0,01$ | 0,5 ± 0,2 |
| Kontrolle | | 2,1 ± 0,2 | 0 |
| Indalpin | 1 | 4,1 ± 0,7 $p < 0,01$ | 2,0 ± 0,7 |

**Analgetischer Test:** Heizplatte

DPPT ist gegen die zwei an der einem Wärmeschmerzstimulus ausgesetzten Maus beobachteten Reaktionen aktiv. Der Effekt ist bei 16 mg/kg statistisch gesichert. Die mit Desipramin, Indalpin und Morphin erhaltenen Ergebnisse sind in Tabelle 5 zum Vergleich ebenfalls angegeben. Der schadenverhütende Effekt von DPPT unterliegt Antagonismus durch Naloxon. Dieses letztere Ergebnis spricht für einen wirklichen analgetischen Effekt der Substanz (Tabelle 5a).

**Tabelle 5 Heizplattentest an der Maus**

| Behandlung | Dosis [mg/kg] | Pfotenlecken | Sprünge |
|---|---|---|---|
| Kontrolle | | 5,1 ± 0,3 | 90 ± 7 |
| DPPT | 16 | 8,7 ± 1,2 $p < 0,02$ | 154 ± 11 $p < 0,001$ |
| Desipramin | 8 | 8,2 ± 0,5 $p < 0,001$ | 85 ± 7 |
| Indalpin | 8 | 8,4 ± 1,2 $p < 0,02$ | 132 ± 12 p ± 0,01 |
| Kontrolle | | 6,3 ± 0,7 | 96 ± 9 |
| DPPT | 8 | 7,0 ± 0,9 | 103 ± 10 |
| | 16 | 9,9 ± 2,0 | 135 ± 14 $p < 0,05$ |
| | 32 | 13,0 ± 1,7 $p < 0,01$ | 193 ± 17 $p < 0,001$ |
| Kontrolle | | 5,5 ± 0,4 | 86 ± 10 |
| Morphin | 2 | 8,1 ± 0,6 $p < 0,01$ | 158 ± 17 $p < 0,01$ |

**Tabelle 5a Heizplattentest an der Maus**
**Wechselwirkung mit Naloxon**

| Behandlung | Dosis [mg/kg] | Pfotenlecken | Sprünge | |
|---|---|---|---|---|
| Kontrolle | | 5,3 ± 0,3 | 84 ± 10 | |
| Naloxon | 0,5 | 5,5 ± 0,3 | 63 ± 6 | |
| DPPT | 16 | 10,0 ± 1,5 p < 00,1 | 141 ± 12 p < 0,01 | p < 0,05 |
| DPPT<br>Naloxon | 16<br>0,5 | 7,1 ± 0,5 p < 0,01 | 98 ± 12 n.s. | |
| Kontrolle | | 5,9 ± 0,5 | 78 ± 6 | |
| Naloxon | 0,5 | 5,2 ± 0,3 | 67 ± 5 | |
| DPPT | 16 | 7,9 ± 0,6 p < 0,02 | 125 ± 14 p < 0,01 | p < 0,05 |
| DPPT<br>Naloxon | 16<br>0,5 | 6,5 ± 0,7 n.s. | 88 ± 10 n.s. | |

Als nichteinschränkende Beispiele werden typtische Arbeitsweisen zur Herstellung und die physikalisch-chemischen Kenndaten der neuen Verbindungen im folgenden beschrieben:

**BEISPIEL 1:**

**Herstellung von cis-4-(4-Methoxyphenyl)-3-phenylpiperidin 1**

Man gibt 2 g (0,071 Mol) cis-4-(4-Methoxyphenyl)-3-phenylpiperidon-2 portionsweise im Verlauf von 15 Minuten in einem 1 g (0,026 Mol) Lithiumaluminiumhydrid und 60 $cm^3$ wasserfreien Äther enthaltenden Kolben und hält unter Rühren und unter Stickstoff bei -10°C.

Man läßt das Reaktionsgemisch 1 Stude bei Raumtemperatur stehen und hydrolysiert dann bei -5°C mit 100 $cm^3$ Wasser.

Der Äther wird abgedampft und der Rückstand mit Methylenchlorid (4 x 30 $cm^3$) extrahiert und über wasserfreiem Natriumsulfat getrocknet.

Nach Eindampfen erhält man quantitativ ein viskoses Gemisch, das ungefähr 80 % des Piperidins 1 enthält. Dieses wird durch Chromatographie über Kieselsäure gereinigt.

"Flash"-Chromatographie: Säule mit Kieselsäure 230-400 mesh ASTM, 25 cm und 4 cm Durchmesser. Durchflußmenge 13,5 $cm^3$/min. Laufmittel Essigester/Methanol 40/60. Rf = 0,27.

Nach Abdampfen der Lösungsmittel erhält man 1,42 g (74%) einer weißen hygroskopischen, dem reinen cis-1-Racemat entsprechenden Verbindung. Schmelzpunkt = 69-70°C.
Chlorhydrat: Schmelzpunkt = 203-204°C (absolutes Äthanol).

**Analyse:** $C_{18}H_{21}NO$

| | C | H | N | O |
|---|---|---|---|---|
| Berechnet mit 0,16 Mol Wasser pro Mol Piperidin | 80,01 | 7,89 | 5,18 | 6,90 |
| Gefunden | 79,76 | 7,93 | 5,13 | 6,65 |

**IR(KBr)** (Base) : ν N-H bei 3330 $cm^{-1}$, schwach.
**Massenspektrometrie (Base) :** (70ev); m/z (relative Intensität):
267(77:M⁺), 247(17), 176(28), 196(38), 134(28), 133(47), 104(100), 91(19), 77(21), 57(32).
**NMR 80 MHz** (Base) $CDCl_3$:
7,35-6,95 ppm (5H, Ar, Komplex)
6,90-6,55 ppm (4H, Ar, Komplex)
3,70 ppm (3H, s, Methoxy)
3,50-2,70 ppm (6H, breiter Komplex)
2,40-1,45 ppm (3H, breiter Komplex einschließlich des N-H-Singletts bei 1,82 ppm).
**NMR 350 MHz (Chlorhydat)** $CDCl_3$:
10,10-9,80 ppm (2H, $\overset{+}{N}H_2$, Komplex)
7,22-7,10 ppm (3H, Ar, Komplex)
6,95-6,86 ppm (2H, Ar, Komplex)
6,82 ppm (2H, Ar, d., Jortho-meta = 8,4 Hz)
6,68 ppm (2H, Ar, d., Jortho-meta = 8,4 hz)
3,80-3,70 ppm (4H, Komplex mit Methoxy-Singlett bei 3,75 ppm)
3,63-3,40 ppm (5H, Komplex)
2,63-2,47 ppm (1H, Komplex)
2,36-2,24 ppm (1H, Komplex)

**BEISPIEL 2:**

**Herstellung von trans-4-(4-Methoxyphenyl)-3-phenylpiperidinchlorhydrat 2**

Man gibt 2 g (0,071 Mol) trans-4-(4-Methoxyphenyl-3-phenylpiperidon-2, 0,65 g (0,017 Mol) Lithiumaluminiumhydrid und 30 $cm^3$ wasserfreies Dioxan unter Rühren und in einer Stickstoffatmosphäre in einen 100 $cm^3$-Kolben.

Nach 1 Stunde unter Rückfluß hydrolysiert man zwischen 0°C und 5°C mit 10 $cm^3$ Wasser.

Der nach Abdampfen des Lösungsmittels erhaltene feste Rückstand wird mit Methylenchlorid (6 x 15 $cm^3$) extrahiert und die organische Phase über wasserfreiem Natriumsulfat getrocknet.

Nach Filtrieren leitet man 5 Minuten lang gasförmigen Chlorwasserstoff in die organische Phase.

Nach Eindampfen erhält man einen gelben bröckeligen Feststoff, der aus 15 $cm^3$ eines 60/40-Gemischs aus Äther und absolutem Äthanol umkristallisiert wird.

Nach Filtrieren, Waschen mit wasserfreiem Äther und Trocknen erhält man 1,36 g (62 %) eines hygroskopischen kristallisierten Produkts, das dem Chlorhydrat des reinen trans-Racemats 2 entspricht. Schmelzpunkt = 218 - 220°C.

**Analyse:** $C_{18}H_{21}NO.HCl$

| | C | H | N | Cl |
|---|---|---|---|---|
| Berechnet mit 0,6 Mol Wasser pro Mol Chlorhydrat | 68,70 | 7,38 | 4,45 | 11 |
| Gefunden | 68,59 | 7,34 | 4,48 | 11,40 |

**IR(KBr)** (Chlorhydrat):
breite "Ammonium"-Bande, Mittel um 2750 $cm^{-1}$ starke Bande um 2920 $cm^{-1}$ (ν as-$CH_2$-).
Massenspektrometrie (Chlorhydrat): 70 eV; m/z (relative Intensität):
267(78;M⁺), 225(5), 210(7), 176(31), 167(5), 165(8), 159(5), 152(50), 147(8), 134(24), 133(44), 121(14), 104-(100), 91(12), 78(7), 77(8), 57(22), 56(15).
**NMR 350 MHz** (Chlorhydat) $CDCl_3$:
10-9,80 ppm (2H, $\overset{+}{N}H_2$, Komplex)
7,20-6,90 ppm (7H, Ar, Komplex)
6,68 ppm (2H, Ar, d., Jortho-meta = 8,4 MHz)
3,80-3,54 ppm (5H, Komplex mit Methoxy-Singlett bei 3,68 ppm)
3,54-3,40 ppm (1H, Komplex)
3,24-3,04 ppm (2H, Komplex)

3,00-2,85 ppm (1H, Komplex)
2,52-2,36 ppm (1H, Komplex)
2,22-2,02 ppm (1H, Komplex).

**BEISPIEL 3:**

**Herstellung von trans-3-Phenyl-4-(4-trifluormethylphenyl)-piperidin-chlorhydrat 3**

Man gibt 5 g (0,015 Mol) trans-3-Phenyl-4-(4-trifluormethylphenyl)-piperidon-2, 1,5 g (0,039 Mol) Lithiumaluminiumhydrid und 60 $cm^3$ wasserfreies Dioxan unter Rühren in eine Stickstoffatmosphäre.

Nach 45 Minuten am Rückfluß hydrolysiert man zwischen 0°C und 5°C mit 25 $cm^3$ Wasser.

Der nach Abdampfen des Lösungsmittels erhaltene feste Rückstand wird mit Methylenchlorid extrahiert und die organische Phase über wasserfreiem Natriumsulfat getrocknet. Nach Filtrieren und Abdampfen des Lösungsmittels erhält man 4,7 g (98 %) gelbes Öl, das man über Kieselsäure von 230-400 mesh ASTM (Laufmittel: 60/40 Essigester/MeOH) chromatographiert.

Dabei gewinnt man 3,3 g (69 %) öliges Produkt, das dem reinen trans-Racemat entspricht. Nach Anlösen dieses Öls in wenig wasserfreiem Äther wird gasförmiger HCl durchgeperlt.

Nach Abdampfen des Lösungsmittels erhält man 3,6 g (67 %) Chlorhydrat des reinen trans-Piperidins 3 in Form eines bröckeligen Schaums.

**Analyse:** $C_{18}H_{19}ClF_3N$

| | C | H | N | Cl |
|---|---|---|---|---|
| Berechnet mit 0,44 Mol Wasser pro Mol Produkt | 61,81 | 5,72 | 4,00 | 10,12 |
| Gefunden | 61,81 | 6,15 | 3,89 | 9,90 |

**Massenspektrometrie:** (70 eV); m/z (relative Intensität) :
306(8); 305(43,7;M⁺ -HCl); 304(8,6%); 256(5); 167(12); 149(32,7); 137(12,4); 133(9); 132(14); 104(30); 97-(13); 95(13,5); 91(13); 85(14); 81(37); 57(100).

**NMR** $^{13}C$: $CDCl_3$

| δ | $C_2$ | $C_3$ | $C_4$ | $C_5$ | $C_6$ |
|---|---|---|---|---|---|
| Base | 54,58 | 50,23 | 48,92 | 35,50 | 47,03 |
| Chlorhydrat | 49,77 | 45,48 | 46,84 | 30,57 | 44,60 |

**BEISPIEL 4:**

**Herstellung von trans-3-Phenyl-4-(4-trifluormethylphenyl)-piperidin-maleinat 4**

Man gibt 0,55 g (0,0047 Mol) Maleinsäure in 60 $cm^3$ Äther tropfenweise zu 1,1 g (0,0036 Mol) der reinen trans-3-Phenyl-4-(4-trifluormethylphenyl)-piperidinbase aus Beispiel 3, gelöst in 40 $cm^3$ wasserfreiem Äther, und rührt über Nacht. Der erhaltene Niederschlag wird abfiltriert. Dabei erhält man 1 g (66 %), das beim Umkristallisieren aus Essigester/Äthergemisch 0,9 g (59 %) reines Malienat 4 in Form weißer Kristalle liefert. Schmelzpunkt = 146-147°C.

**Analyse:** $C_{22}H_{22}F_3NO_4$

| | C | H | F | N |
|---|---|---|---|---|
| Berechnet | 62,70 | 5,26 | 13,52 | 3,32 |
| Gefunden | 62,60 | 5,27 | 13,27 | 3,25 |

**BEISPIEL 5:**

**Herstellung von trans-4-(4-Chlor-trifluormethylphenyl)-3-phenylpiperidin-chlorhydrat 5**

Man gibt zu einer Lösung von 15 $cm^3$ (0,03 Mol) 2 m-Diboran in THF unter Rühren im Verlauf von 35 Minuten unter Stickstoff bei 0°C 6 g (0,0169 Mol) trans-4-(4-Chlor-3-trifluormethylphenyl)-3-phenylpiperidon-2 in Löusng in 70 $cm^3$ wasserfreiem THF. Die dabei erhaltene farbige Lösung wird dann 2 Stunden lang am Rückfluß erhitzt. Bei Raumtemperatur versetzt man mit 10 $cm^3$ 6 n-HCl, dampft das THF ab und nimmt den Rückstnd in Methylenchlorid auf.

Man gibt Natronlauge bis pH 12 dazu, wäscht neutral und trocknet über wasserfreiem Natriumsulfat.

Nach Eindampfen erhält man 5,2 g (90 %) vikoses Öl, das im wesentlichen das erwartete Piperidin enthält. Nach Reinigung durch Schnellchromatographie über Kieselsäure 230-400 mesh ASTM (60/40 Essigester/Methanol) gewinnt man 4,4 g Piperidinbase, die man zur Herstellung des Chlorhydrats in 60 $cm^3$ wasserfreiem Äther löst.

Dabei erhält man 4,34 g (58 %) bröckeligen Schaum, der dem reinen trans-4-(4-Chlor-3-trifluormethylphenyl)-3-phenylpiperidin-chlorhydrat 5 entspricht.

**Analyse:** $C_{18}H_{18}Cl_2F_3N$

| | C | H | Cl | F | N |
|---|---|---|---|---|---|
| Berechnet mit 0,9 Mol Wasser auf Produkt | 55,10 | 5,07 | 18,07 | 14,52 | 3,56 |
| Gefunden | 55,23 | 5,21 | 17,83 | 14,79 | 3,40 |

**Massenspektrometrie:** (70 eV); m/z (relative Intensität):
342,0(2,8); 340,9(13,4); 340,0(10,1); 339,0(40,6); 338,0(6,1); 178,0(11,2); 133,0(10,7); 132,0(16,0); 104,1-(47,2); 91,0(17,3); 77,0(10,5); 57,1(95,6); 56,1 (55,1); 44,1(100).

**NMR$^{13}$C:** $CDCl_3$

| | $C_2$ | $C_3$ | $C_4$ | $C_5$ | $C_6$ |
|---|---|---|---|---|---|
| δ | 49,48 | 45,43 | 46,36 | 30,20 | 44,39 |

**NMR 350 MHz:** 9,90 ppm (2H, $NH_2$, $\overset{+}{N}H_2$)
7,40-6,90 ppm (8H, Ar, Komplex)
3,74 ppm ($H_{6e}$, m.)
3,64 ppm ($H_{2e}$, m.)
3,53 ppm ($H_{3a}$, m.)
3,19 ppm ($H_{6a}$ + $H_{2a}$, m.)
3,05 ppm ($H_{4a}$, m.)
2,60-2,42 ppm ($H_{5a}$ , m.)
2,11 ppm ($H_{5e}$, m.)

**BEISPIEL 6:**

**Herstellung von trans-4-(4-Chlor-3-trifluormethylphenyl)-3-phenylpiperidin-maleinat 6**

Verfährt man wie in Beispiel 4, so erhält man aus der reinen trans-4-(4-Chlor-3-trifluormethylphenyl)-3-phenylpiperidinbase das Maleinat 6, das beim Umkristallisieren aus Essigester/Metahnolgemisch 80/20 Kristalle liefert. Schmelzpunkt = 185-186°C.

**Analyse:** $C_{22}H_{21}ClF_3NO_4$

| | C | H | Cl | F | N |
|---|---|---|---|---|---|
| Berechnet | 57,95 | 4,61 | 7,79 | 12,51 | 3,07 |
| Gefunden | 58,05 | 4,64 | 7,73 | 12,56 | 3,00 |

**Massenspektrometrie:** (70 eV); m/z (relative Intensität):
342,1(2,2); 341,0(12,5;M$^{\circ}$); 340(8,9); 339,1(36,7; M$^{\circ}$); 338,0(5,9); 266,1(11,5); 248,0(6,6); 193,1(9,7); 181,0-(6,1); 180,0(37,4); 179,0(21,8); 178,0(21,7); 165,1(11,9); 133,1(10,7); 132,0(23,4); 131,0(78,6); 119,0(6,2); 118,0(64,2); 117,1(6,7); 116,1(5,1); 115,1(19,8); 105,1(9,3); 104,1(49,9); 103,0(10,5); 102,0(6,6); 91,0(27,4); 89,9(17,6); 89,0(12,4); 78,0(8,6); 77,0(14,1); 76,0(5,3); 44,1(100).

**BEISPIEL 7:**

**Herstellung von trans-4-(4-Mesyloxyphenyl)-3-phenylpiperidinchlorhydrat 7**

**1. Stufe:** Herstellung von trans -4-(4-Hydroxyphenyl)-3-phenylpiperidin-bromhydrat

Im Verlauf von 1 Stunde 20 Minuten gibt man zu einer Lösung von 9,2 g (0,030 Mol) 2 in 250 $cm^3$ wasserfreiem Methylenchlorid unter Rühren bei -35°C unter Stickstoff eine Lösung von 20,8 g (0,083 Mol; 8 $cm^3$) Bortribromid in 100 $cm^3$ wasserfreiem Methylenchlorid.

Man läßt 4 Stunden bei Raumtemperatur stehen und führt dann die Methanolyse (230 $cm^3$ wasserfreies Methanol) des Produkts bei -35°C durch.

Dann läßt man die Temepratur wieder ansteigen, wobei man zum Austreiben des gebildeten überschüssigen Bromwasserstoffs einen starken Stickstoffstrom durch das Reaktionsgemisch leitet.

Nach 4 Stunden Rühren bei Raumtemperatur wird die Lösung im Vakuum eingeengt (Temperatur ≤ 30°C). Dabei erhält man 8,5 g (84 %) beigefarbigen Feststoff, der aus 95 %igem Äthanol umkristallisiert wird. Gewicht des erhaltenen Produkts: 8 g. Ausbeute 79 %. Schmelzpunkt = 263-264°C.

**Analyse:** $C_{17}H_{20}BrNO$

| | C | H | N | Br |
|---|---|---|---|---|
| Berechnet | 61,08 | 6,03 | 4,19 | 23,90 |
| Gefunden | 60,92 | 6,08 | 4,06 | 23,84 |

**IR(KBr):** $\nu$ N-H bei 3260 $cm^{-1}$, mittelstark.
**Massenspektrometrie:** (70 eV); m/z (relative Intensität):
253,1(100;M$^{\circ}$); 196(14); 164(10); 162(31); 133(40); 132(15); 120(19); 107(13); 105(15); 104(92); 103(11); 91-(15); 82(23); 81(11); 80(23); 79(11); 77(13); 57(36); 56(23).

**2. Stufe:**Herstellung von N-Benzyloxycarbonyl-trans-4-(4-hydroxyphenyl)-3-phenylpiperidin

Man rührt ein Gemisch aus 6,5 g (0,019 Mol) trans-4-(4-Hydroxyphenyl)-3-phenylpiperidin-bromhydrat und 7,9 g (0,039 Mol) Natriumtetraborat in 100 $cm^3$ Wasser bei Raumtemperatur.

Während des Einlaufens von 5 g (0,029 Mol) Chlorameisensäurebenzylester im Verlauf von 15 Minuten hält man den pH des Reaktionsgemischs mittels wäßriger 2 n-Natronlauge auf 9.

Man rührt 45 Minuten bei Raumtemperatur und neutralisiert mit verdünnter Salzsäure.

Man extrahiert mit Methylenchlorid (5 x 80 $cm^3$), wäscht die organische Phase mit Wasser und trocknet über wasserfreiem Natriumsulfat.

Nach Eindampfen gewinnt man quantitativ 8 g rötlich-gelbes Öl, das beim Kristallisieren aus Äther 5,8 g weißes kristallisiertes Produkt ergibt, das dem erwarteten Produkt entspricht. Ausbeute = 77 %.

**3. Stufe:** Herstellung von N-Benzyloxycarbonyl-trans-4-(4-mesyloxyphenyl)-3-phenylpiperidin.

Zu 2 g (0,005 Mol) N-Benzyloxycarbonyl-trans-4-(4-hydroxyphenyl)-3-phenylpiperidin in 12 $cm^3$ wasserfreiem Pyridin gibt man bei 0°C unter Stickstoff tropfenweise 1,2 g (0,01 Mol) Mesylchlorid.

Man rührt 2 Stunden lang bei dieser Temperatur und stellt das Reaktionsgemisch dann für 24 Stunden in einem Kühlschrank. Danach wird es in ein 50 g Eis 13 $cm^3$ konzentrierte Salzsäure enthaltendes Becherglas gegossen.

Man extrahiert mit Methylenchlorid (4 x 80 $cm^3$), wäscht die organische Phase neutral (2 x 30 $cm^3$) und trocknet über wasserfreiem Natriumsulfat.

Nach Eindampfen erhält man 2,15 g (89 %) eines gelblichen Schaums.

Das isolierte Produkt wird durch "Flash"-Chromatographie gereinigt. Kieselgel 230-240 mesh. Laufmittel: Hexan/Essigester 60/40. Dabei gewinnt man 1,7 g des erwarteten Produkts. Ausbeute: 71%.

**4. Stufe:** Herstellung von <u>7</u>

In einem Autoklaven rührt man 2,3 g (0,005 Mol) N-Benzyloxycarbonyl-trans-4-(4-mesyloxyphenyl)-3-phenylpiperidin, 0,4 g 10 %ige Palladiumkohle und 40 $cm^3$ Eisessig 3 Stunden lang bei Raumtemperatur unter 10 bar Wasserstoffdruck.

Nach Filtrieren und Eindampfen nimmt man das dabei erhaltene Öl in 120 $cm^3$ Methylenchlorid auf, wäscht dann die organische Phase mit Wasser neutral und trocknet schließlich über wasserfreiem Natriumsulfat.

Nach Eindampfen gewinnt man quantitativ ein organgefarbenes Öl, von dem man das Chlorhydrat herstellt.

Das dabei gewonnene Chlorhydrat wird durch "Flash"-Chromatographie gereinigt. Kieselgelsäule 230-240 mesh. Laufmittel: Essigester/Methanol 60/40 und dann reines Methanol. Dabei erhält man 0,9 g weißen Feststoff, der sich nicht umkristallisieren läßt und dem erwarteten Produkt 7 entspricht. Ausbeute 50 %.

**Massenspektrometrie:** (Chlorhydrat) (70 eV); m/z (relative Intensität):
331(5;$M^{\oplus}$); 104(10); 91(6); 74(66), 60(38), 59(100).

**NMR ($^{13}C$): $CDCl_3$; ppm:**

| | $C_2$ | $C_3$ | $C_4$ | $C_5$ | $C_6$ | $C_3$ |
|---|---|---|---|---|---|---|
| δ | 49,77 | 45,82 | 46,75 | 30,59 | 44,65 | 37,52 |

**BEISPIEL 8:**

**Herstellung von trans-3-(4-Chlorphenyl)-4-phenylpiperidin 8**

Man gibt 1,2 g (0,004 Mol) 3-(4-Chlorphenyl)-4-phenylpiperidon-2 in Lösung in 20 $cm^3$ wasserfreiem Dioxan mit 0,4 g (0,01 Mol) Lithiumaluminiumhydrid in einen Kolben und rührt unter einer Stickstoffatmosphäre. Im Verlauf 1 Stunde erhitzt man das Reaktionsgemisch langsam bis zum Rückfluß des Dioxans und hält diesen 1 Stunde 15 Minuten lang aufrecht. Während der Hydrolyse kühlt man das Reaktionsgemisch auf 0°C bis 5°C und versetzt mit 20 $cm^3$ Methylenchlorid. Nach Abdampfen der Lösungsmittel wird der Rückstand mit Methylenchlorid (4-mal 20 $cm^3$) extrahiert und die organische Phase mit Wasser gewaschen und über wasserfreiem Natriumsulfat getrocknet.

Nach Filtrieren und Abdampfen des Lösungsmittels erhält man 1,17 g Produkt, welches des trans-Piperidin 8 und ungefähr 20 % Verunreinigungen enthält. Dieses Gemisch wird durch Flash-Chromatographie gereinigt:
Säule mit Kieselgel 60 (230-400 mesh ASTM),
Essigester/Methanol: 60/40,
Stickstoffdruck: 0,2 bar.

Dies liefert 1,05 g trans-Piperidin 8 vom Schmelzpunkt = 55-58°C.

Das in Form eines bröckeligen Schaums erhaltene Chlorhydrat läßt sich nicht umkristallisieren.

**IR-(KBr):** ν N-H bei 3220 $cm^{-1}$, schwach.

**Massenspektrometrie:** (70 eV); m/z (relative Intensität):
273(36,$M^{0}$); 271(100,$M^{0}$); 273(16); 179(22); 178(35); 166(16); 146(30); 140(20); 138(53); 115(17); 104(21); 103(23); 91(29); 86(18); 84(27); 77(23).

**BEISPIEL 9:**

**Herstellung von cis-(4-(4-Methylphenyl)-3-phenylpiperidin 9**

Man gibt 8 g cis-4-(4-Methylphenyl)-3-phenylpiperidon portionsweise im Verlauf von 15 Minuten unter Rühren und unter einer Stickstoffatmosphäre in einem 4,5 g (0,118 Mol) Lithiumaluminiumhydrid in 140 $cm^3$ wasserfreiem Äther enthaltenden Kolben und läßt dann die Reaktion 1 Stunde lang bei Raumtemperatur ablaufen. Nach der Behandlung erhält man 5,23 g (69 %) reines cis-Piperidin 9 vom Schmelzpunkt = 55-57°C.

**Analyse: $C_{18}H_{21}N$**

| | C | H | N |
|---|---|---|---|
| Berechnet | 86,01 | 8,41 | 5,57 |
| Gefunden | 85,71 | 8,36 | 5,50 |

**IR(KBr):** ν N-H bei 2320 $cm^{-1}$, schwach.

**Massenspektrometrie:** (70 eV); m/z (relative Intensität):
251(100, $M^{0}$); 208(12); 178(11); 160(30); 159(11); 133(27); 132(27); 118(16); 117(18); 115(12); 105(20); 104-(68); 103(12); 91(18); 77(12); 57(36); 56(27).

Das Chlorhydrat des cis-4-(4-Methylphenyl)-3-phenylpiperidins wird auf übliche Weise hergestellt. Es wird aus Methylenchlorid/Hexangemisch umkristallisiert. Schmelzpunkt = 227-230°C.

**BEISPIEL 10:**

**Herstellung von trans-4-(4-Methylphenyl)-3-phenylpiperidin 10**

Man reduziert 7,5 g des entsprechenden trans-Piperidons nach der üblichen Arbeitsweise. Dies ergibt quantitativ das trans-Piperidin 10. Umkristallisieren aus einem ternären Äther/Hexan/Methylenchloridgemisch (60/30/10) liefert Kristalle des reinen trans-Piperidins 10 mit Schmelzpunkt = 90-93°C.

**Analyse: $C_{18}H_{21}N$**

| | C | H | N |
|---|---|---|---|
| Berechnet (0,22 Mol $H_2O$) | 84,68 | 8,45 | 5,48 |
| Gefunden | 84,68 | 8,49 | 5,43 |

**IR(KBr):** ν N-H bei 3340 $cm^{-1}$, mittelstark

**Massenspektrometrie:** (70 eV); m/z (relative Intensität):
251(100,$M^{0}$); 208(13); 194(8); 179(11); 178(13); 160(32); 159(11); 133(26); 132(18); 131(10); 118(13); 117-(10); 115(11); 105(18); 104(63); 91(4); 77(8); 57(40); 56(29).

Das N-Benzoylderivate des trans-Piperidins 10 wird auf übliche Weise hergestellt und aus absolutem Äthanol/Äther 60/40 umkristallisiert, wobei man Kristalle vom Schmelzpunkt = 137,5-138,5°C erhält.

Das auf übliche Weise hergestellte Chlorhydrat des trans-Piperidins 10 wird aus Methylenchlorid umkristallisiert und liefert Kristalle vom Schmelzpunkt = 253-254°C.

**BEISPIEL 11:**

**Hestellung von trans-4-(4-Acetoxyphenyl)-3-phenylpiperidinchlorhydrat 11**

1.) Zu 10,8 g (0,027 Mol) N-Benzyloxycarbonyl-trans-4-(4-hydroxyphenyl)-3-phenylpiperidin (in Beispiel 7 beschriebene Verbindung), gelöst in 50 $cm^3$ wasserfreiem Pyridin, gibt man unter Rühren bei 7°C und unter Stickstoff 5,5 $cm^3$ (0,077 Mol) Acetylchlorid. Man rührt 7 Minuten lang bei 7°C und dann 48 Stunden lang bei Raumtemperatur. Das Reaktionsgemisch wird auf 250 g Eis gegossen und mit Methylenchlorid (4-mal 100 $cm^3$) extrahiert. Die organische Phase wird mit verdünnter Salzsäure auf sauren pH und mit Wasser neutral gewaschen und dann über wasserfreiem Natriumsulfat getrocknet. Nach Filtrieren und Eindampfen gewinnt man quantitativ einen braunen Schaum, der in der Mindestmenge Essigester gelöst wird, und filtriert dann die so erhaltene Lösung über Kieselgel 230-400 mesh ASTM. Das Lösungsmittel wird abgedampft und der so erhaltene Rückstand aus 95 %igem Äthanol umkristallisiert, wobei man 7,6 g (63 %) N-Benzyloxycarbonyl-trans-4-(4-acetoxyphenyl)-3-phenylpiperidin in Form weißer Kristalle von Schmelzpunkt = 120°C erhält.

2.) Man gibt 3 g (0,007 Mol) der obigen Verbindung, 50 $cm^3$ Eisessig und 0,5 g Palladiumkohle (10 % Pd/C) in einen 250 $cm^3$ Autoklaven und hydriert unter Rühren 4 Stunden lang bei Raumtemperatur unter 10 bar Wasserstoffdruck. Nach Filtrieren und Abdampfen des Lösungsmittels erhält man ein Öl, das man in Methylenchlorid zur Lösung bringt. Nach Einleiten gasförmigen Chlorwasserstoffs und Abdampfen des Lösungsmittels erhält man einen breiigen Schaum, der aus Äthanol/Äthergemisch kristallisiert wird, wobei man 1,9 g (82 %) Chlorhydrat 11 vom Schmelzpunkt = 198°C (Änderung des Aussehens um 193°C) erhält.

**Analyse:** $C_{19}H_{22}ClNO_2$

| | C | H | N | Cl |
|---|---|---|---|---|
| Berechnet (0,43 Mol $H_2O$/Mol Produkt): | 67,20 | 6,78 | 4,12 | 10,44 |
| Gefunden | 67,18 | 6,79 | 3,98 | 10,60 |

**IR(KBr):** ν C=O bei 1750 $cm^{-1}$, stark, N zwischen 2640 und 2820 $cm^{-1}$, stark.

**Massenspektrometrie:** (70 eV); m/z (relative Intensität):
269,1(18,1); 294,9(75,8,M°); 253,0(5,4); 204,0(21,4); 196,0(9,9); 165,0(5,1); 162,0(9,5); 135,0(6,6); 133,0-(29,6); 132,0(18,0); 121,0(5,5); 119,9(21,2); 115,0(5,2); 107,0(9,3); 105,1(13,4); 104,0(100,0); 103,0(7,5); 91,0-(12,9); 77,0(8,1); 57,1(80,1); 56,1(33,9).

NMR ($^{13}C$): ($CDCl_3$): Signale δ in ppm

| $C_2$ | $C_3$ | $C_4$ | $C_5$ | $C_6$ | $C_6H_3$ |
|---|---|---|---|---|---|
| 49,64 | 45,72 | 46,50 | 30,57 | 44,55 | 21,07 |

**BEISPIEL 12:**

**Herstellung von cis-4-(4-Chlorphenyl)-3-phenylpiperidin 12**

Man gibt 2 g (0,007 Mol) 4-(4-Chlorphenyl)-3-phenylpiperidone-2 im Verlauf von 15 Minuten unter Rühren bei 15°C unter einer Stickstoffatmosphäre in einen 1 g (0,026 Mol) Lithiumaluminiumhydrid in 30 $cm^3$ wasserfreiem Äther enthaltenden Kolben. Nach beendeter Zugabe läßt man die Reaktion 30 Minuten

lang ablaufen und hydrolysiert dann bei -5°C durch tropfenweisen Zusatz von 8 $cm^3$ Wasser, versetzt danach mit 50 $cm^3$ Methylenchlorid und stellt den pH auf 7 ein. Nach Abdampfen der Lösungsmittel extrahiert man den Rückstand mit Methylenchlorid (4-mal 25 $cm^3$) und trocknet die organische Phase über wasserfreiem Natriumsulfat. Nach Filtrieren liefert Abdampfen des Lösungsmittels ein Rohprodukt, das aus wasserfreiem Äther/Hexangemisch 60/40 umkristallisiert wird und dabei 1,2 g (63 %) des sehr hygroskopischen cis-Produkts 12 vom Schmelzpunkt= 63-65°C ergibt. Das N-Benzoylderivat besitzt einen Schmelzpunkt= 152-153°C.

**Analyse:** $C_{24}H_{12}ClNO$

| | C | H | Cl | N | O |
|---|---|---|---|---|---|
| Berechnet (0,13 Mol $H_2O$/Mol Produkt): | 76,21 | 5,92 | 9,37 | 3,70 | 4,78 |
| Gefunden | 76,21 | 5,84 | 9,22 | 3,71 | 4,42 |

**IR(KBr):** ν N-H bei 2320 $cm^{-1}$, schwach (Produkt 12).

**Massenspektrometrie:** (70 eV); m/z (relative Intensität), (Produkt 12):
273,1(36,M⁺); 271,0(100,M⁺); 237(19); 180(24); 179(18); 178(18); 133(22); 132(25); 115(12); 105(12); 104-(85); 103(21); 91(16); 78(12); 77(15); 15(72); 56(46).

**BEISPIEL 13:**

**Herstellung von trans-3-(3,4-Dichlorphenyl)-4-phenyl-piperidin-maleinat 13**

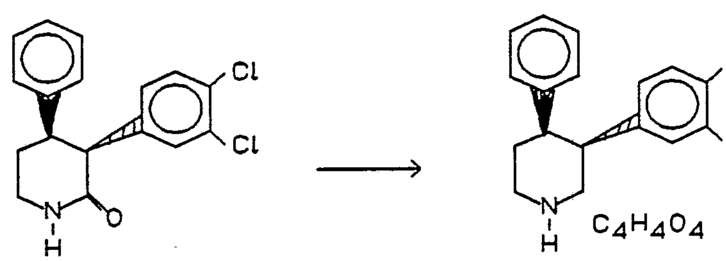

Zu 6 g (0,01874 Mol) trans-3-(3,4-Dichlorphenyl)-4-phenylpiperidon-2 in Lösung in 35 $cm^3$ wasserfreiem THF gibt man unter einer Stickstoffatmosphäre bei 0°C im Verlauf von 25 Minuten eine Lösung von 14,2 $cm^3$ (0,0313 Mol) 2,20-m Boran in THF.

Die erhaltene Lösung wird 2 Stunden lang am Rückfluß erhitzt. Nach Wiederabkühlung auf Raumtemperatur gibt man 5 $cm^3$ 6-n Salzsäure dazu, dampft das THF ab und löst den Rückstand in 100 $cm^3$ Dichlormethan.

Man setzt 10 $cm^3$ Natronlauge dazu, trennt die organische Phase ab, wäscht sie mit Wasser neutral und trocknet über wasserfreiem Natriumsulfat.

Nach Filtrieren und Eindampfen erhält man 5,4 g (94 %) viskoses Öl, das 90 % des erwarteten Piperidins enthält (analytische Hochdruckflüssigkeitschromatographie, Partisil-10-Säule, Laufmittel: Essigester/Methanol/$NH_4Br$ 60/40/0,02 m).

Das so gewonnene Piperidin wird direkt in das Maleinat überführt.

**Maleinat:** Man versetzt eine Lösung von 2,73 g (0,0235 Mol) Maleinsäure in 200 $cm^3$ Äther unter Rühren mit dem rohen Piperidin, in 150 $cm^3$ Äther gelöst, und läßt 1 Stunde lang bei Raumtemperatur stehen.

Nach Abdampfen des Äthers wird das rohe Maleinat aus absolutem Äthanol umkristallisiert. So gewinnt man 2,85 g (36 %) des reinen trans-Piperidins 3. Schmelzpunkt= 162-164°C.

**Analyse:** $C_{21}H_{21}Cl_2NO_4$

| | C | H | Cl | N |
|---|---|---|---|---|
| **Berechnet %** | 59,73 | 5,01 | 16,79 | 3,32 |
| **Gefunden %** | 59,47 | 5,06 | 16,92 | 3,30 |

**IR(KBr):**
ν≡N 1615 $cm^{-1}$, mittelstark,
ν COOθ1560 $cm^{-1}$, mittelstark, 1360, stark,
ν CH=CH (cis) 695 $cm^{-1}$, stark.

**Massenspektrometrie:** (70 eV); m/z (relative Intensität): 309(12,5,$M^{\oplus}$-116); 307(61,1 $M^{\oplus}$-116); 305($M^{\oplus}$-116); 212(12,5); 202(13,2); 201(12,5); 200(14); 178(23); 176-(14); 174(30,5); 172(43); 146(32,5); 119(10); 117(10,5); 115(12); 104(15); 103(11); 102(12); 99(10,5); 91(21,5); 77(12); 72(41); 57(100); 56(58).

In der nachfolgenden Tabelle 6 sind die in Beispiel 1 bis 13 bereits beschriebenen Piperidine sowie weitere erfindungsgemäße, der allgemeinen Formel I entsprechende Piperidine angegen, die auf den oben beschriebenen Beispielen analoge Arbeitsweise synthetisiert wurden.

TABELLE 6

| Produkt | X | Y | Stereochemie | Base, Fp.(°C) Lsgm. | Salz, Fp. (°C) Lsgm. oder N-Benzoylderivate |
|---|---|---|---|---|---|
| 1 | $4-CH_3O$ | H | cis | 69-70 | HCl, 203-204 Äthanol |
| 2 | $4-CH_3O$ | H | trans | - | HCl, 218-220 Äther/Äthanol 60/4( |
| 3 u. 4 | $4-CF_3$ | H | trans | - | (4) Maleinat 146-147 EE*/Äther |
| 5 u. 6 | 4-Cl $3-CF_3$ | H | trans | - | (6) Maleinat, 185-186 EE/MeOH 80/20 |
| 7 | 4-HO ** | H | trans | - | HBr, 263-264 Äthanol/Wasser |
| | $4-CH_3SO_3$*** | H | trans | - | HCl, weißer Feststoff |
| 8 | H | 4-Cl | trans | 55-58 | HCl |
| 9 | $4-CH_3$ | H | cis | 55-57 | HCl, 227-230 $CH_2Cl_2$/Hexan |
| 10 | $4-CH_3$ | H | trans | 90-93 $Et_2O$/ Hex./ $CH_2Cl_2$ (6/3/1) | HCl, 253-254 $CH_2Cl_2$ N-Benzoylderivate 137,5-138,5 Äthanol/Äther 60/40 |
| 11 | $4-CH_3-\underset{\underset{O}{\|}}{C}-O$ | H | trans | - | HCl, 198 Äthanol/Äther |
| 12 | 4-Cl | H | cis | 63-65 | N-Benzoylderivat 152-153 |
| 13 | H | 3,4-diCl | trans | - | Maleinat, 162-164 Äthanol |
| 14 | 3,4-diCl | H | cis | - | HCl, 194-194,5 Äthanol/Wasser |
| 15 | 3,4-diCl | H | trans | 68 | HCl, 265 Äthanol/Wasser |
| 16 | H | 4-Cl | cis | 125-126 | HCl, 172 Äthanol/Wasser |
| 17 | H | 3,4-di(OH) | cis | | HBr, 274-276 Äthanol |

EE* = Essigester
** = 1. Stufe
*** = 4. Stufe

TABELLE 6 (Fortsetzung)

| Produkt | X | Y | Stereochemie | Base, Fp.(°C) Lsgm. | Salz, Fp. (°C) Lsgm. oder N-Benzoylderivate |
|---|---|---|---|---|---|
| 18 | 4-Cl | H | trans | | HCl, 289-291 Äthanol |
| 19 | 4-$CF_3$ | H | cis | | HCl, 176-178 |
| 20 | 4-Benzyloxy | H | trans | | HCl, 242-243 absolutes Äthanol |
| 21 | H | H | trans | 117 wasserfreier Äther | HCl, 234-235 |
| 22 | H | 3,4-di(OH) | trans | | HBr, 255-256 Äthanol/Äther 50/50 |
| 23 | 4-O-$C_6H_5$ | H | trans | | HCl, 216-217 Methanol |
| 24 | 4-Et-O | H | trans | | HCl 187-188 Methanol/Äther |
| 25 | 4-$(CH_3)_2N$ | H | trans | | di(HCl), 212-215 Äthanol/Äther (Zersetzung) |

Wie sich aus dem vorhergehenden und Tabelle 6 ergibt, ist die Erfindung keineswegs auf jene Ausbildungsformen, Durchführungen und Anwendungen beschränkt, die mehr im einzelenen beschrieben wurden, sondern umfaßt auch sämtliche Varinaten, auf die ein Fachmann kommen kann, ohne om Umfang und Sinn der vorliegenden Erfindung abzuweichen.

**BEISPIEL 26:** Kapseln

Kapseln, die für die orale Verabreichung geeignet sind enthalten die nachfolgend genannten Bestandteile und können auf an sich bekannte Weise hergestellt werden, indem man Wirk-und Hilfsstoffe vermischt und in Gelatinekapseln abfüllt.

| Bestandteile (pro Kapsel) | Gewicht (mg) |
|---|---|
| DPPT | 50 mg |
| Milchzucker | 100 mg |
| Maisstärke | 30 mg |
| Talkum | 3 mg |
| Kolloidales Siliziumdioxid | 3 mg |
| Magnesiumstearat | 2 mg |

**Beispiel 27:** Kapseln

Es wurden Kapseln folgender Zusammensetzung durch Vermischen von Wirk-und Hilfsstoffen und Abfüllen in Gelatinekapseln hergestellt.

| Bestandteile (pro Kapsel) | Gewicht (mg) |
|---|---|
| Verbindungen gemäß Beispiel 2, 3, 10 oder 12 | 100 mg |
| Mikrokristalline Cellulose | 100 mg |
| Maisstärke | 50 mg |
| Talkum | 5 mg |
| Kolloidales Siliziumdioxid | 2 mg |
| Magnesiumstearat | 3 mg |

**Ansprüche**

1. Arzneimittel, dadurch gekennzeichnet, daß es in Form des Racemats oder eines Enantiomeren ein cis-und/oder trans-3,4-Diphenylpiperidinderivat der Formel (Ia)

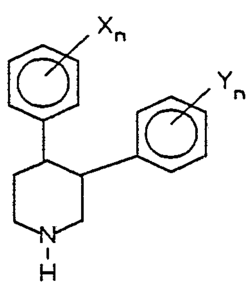

Ia

worin
X und Y gleich oder verschieden sind und für Wasserstoff, Halogen, $C_1$-$C_4$-Alkyl, $C_5$-$C_6$-Cycloalkyl, Phenyl-($C_1$-$C_3$)-alkyl, Trifluormethyl, Trichlormethyl, Hydroxy, $C_1$-$C_4$-Alkoxy, Halogen-($C_1$-$C_4$)-alkoxy, Phenyloxy, Phenyl-($C_1$-$C_3$)-alkoxy, Acyloxy mit bis zu 4 Kohlenstofffatomen, Amino, Nitro, N,N-Dialkylamino mit jeweils 1 bis 4 Kohlenstoffatomen in den Alkylresten, Morpholino oder $C_1$-$C_4$-Alkylsulfonyl stehen und
n eins, zwei oder drei bedeutet,
oder ein physiologisch verträgliches Salz dieser Verbindungen enthält.

2. Antidepressivum gemäß Anspruch 1.

3. Verwendung eines 3,4-Diphenylpiperidinderivates wie in Anspruch 1 definiert als Antidepressium.

4. Arzneimittel gemäß Anspruch 1, dadrurch gekennzeichnet, daß es trans-4-(4-Methylphenyl)-3-phenylpiperidin, trans-4-(4-Methoxyphenyl)-3-phenylpiperidin, trans3-phenyl-4-(4-trifluormethylphenyl)-piperidin oder cis-4-(4-Chlorphenyl)-3-phenylpiperidin oder ein physiologisch verträgliches Salz dieser Verbindungen enthält.

5. Verwendung eines cis-oder trans-3,4-Diphenylpiperidinderivats der im Anspruch 1 angegebenen allgemeinen Formel (Ia) in Form des Racemats oder eines Enantiomeren oder eines Salzes dieser Verbindungen bei der Herstellung eines Arzneimittels mit antidepressiver Wirkung.

6. Verwendung von trans-3,4-Diphenylpiperidin bei der Herstellung eines Arzneimittels mit antidepressiver Wirkung.

7. Substituierte cis-und trans-3,4-Diphenylpiperidinderivate der Formel I

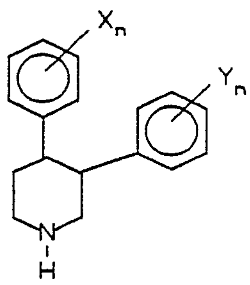

I

worin
X und Y gleich oder verschieden sind und für Halogen, $C_1$-$C_4$-Alkyl, $C_5$-$C_6$-Cycloalkyl, Phenyl-($C_1$-$C_3$)alkyl, Trifluormethyl, Trichlormethyl, Hydroxy, $C_1$-$C_4$-Alkoxy, Halogen-($C_1$-$C_4$)-alkoxy, Phenyloxy, Phenyl($C_1$-$C_3$)-alkoxy, Acyloxy mit bis zu 4 Kohlenstofffatomen, Nitro, N,N-Dialkylamino mit jeweils 1 bis 4 Kohlenstoffatomen in den Alkylresten, Morpholino oder $C_1$-$C_4$-Alkylsulfonyl stehen oder einer der Reste X oder Y Wasserstoff darstellt, und
n eins, zwei oder drei bedeutet,
oder ein physiologisch verträgliches Salz dieser Verbindungen, ausgenommen Verbindungen, worin einer der Substituenten X oder Y $C_1$-$C_4$-Alkoxy und der andere Wasserstoff und n gleich 2 ist.

8. Verbindungen gemäß Anspruch 7, dadurch gekennzeichnet, daß sie in Form eines Enantiomeren vorliegen.

9. Verfahren zur Herstellung von Verbindungen nach Anspruch 7, dadurch gekennzeichnet, daß man ein Reduktionsmittel für die Amidfunktion eines Piperidons auf ein Piperidon der Formel II

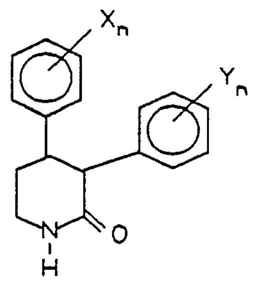

II

worin X, Y und n die zu Formel I angegebenen Bedeutungen haben, einwirken läßt, um das entsprechende Piperidin I derselben cis-oder trans-Stereochemie wie des Ausgangspiperidons zu erhalten, und das Reaktionsprodukt gegebenenfalls in ein physiologisch verträgliches Salz überführt.

Patentansprüche für die folgenden Vertragsstaaten : GR, ES.

1. Verfahren zur Herstellung eines Arzneimittels, dadurch gekennzeichnet, ein cis-und/oder trans-3,4-Diphenylpiperidinderivat der Formel (Ia)

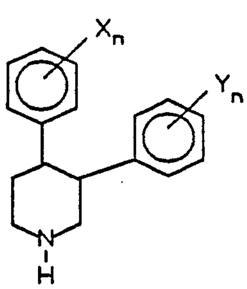

Ia

worin
X und Y gleich oder verschieden sind und für Wasserstoff, Halogen, $C_1$-$C_4$-Alkyl, $C_5$-$C_6$-Cycloalkyl, Phenyl-($C_1$-$C_3$)-alkyl, Trifluormethyl, Trichlormethyl, Hydroxy, $C_1$-$C_4$-Alkoxy, Halogen-($C_1$-$C_4$)-alkoxy, Phenyloxy, Phenyl-($C_1$-$C_3$)-alkoxy, Acyloxy mit bis zu 4 Kohlenstofffatomen, Amino, Nitro, N,N-Dialkylamino mit jeweils

1 bis 4 Kohlenstoffatomen in den Alkylresten, Morpholino oder $C_1$-$C_4$-Alkylsulfonyl stehen und
n eins, zwei oder drei bedeutet,
oder ein physiologisch verträgliches Salz dieser Verbindung, in Form des Racemats oder eines Enantiomeren in eine geeignete Darreichungsform überführt.

2. Verfahren gemäß Anspruch 1, dadurch gekennzeichnet, daß man ein Antidepressivum enthaltend eien Verbindung der Formel Ia oder ein Salz dieser Verbindungen herstellt.

3. Verfahren gemäß Anspruch 1, dadurch gekennzeichnet, daß man trans-4-(4-Methylphenyl)-3-phenylpiperidin, trans-4-(4-Methoxyphenyl)-3-phenylpiperidin, trans-3-Phenyl-4-(4-trifluormethylphenyl)-piperidin oder cis-4-(4-Chlorphenyl)-3-phenylpiperidin oder ein physiologisch verträgliches Salz dieser Verbindungen in eine geeignete Darreichungsform überführt.

4. Verwendung eines cis-oder trans-3,4-Diphenylpiperidinderivats der im Anspruch 1 angegebenen allgemeinen Formel (Ia) in Form des Racemats oder eines Enantiomeren oder eines Salzes dieser Verbindungen bei der Herstellung eines Arzneimittels mit antidepressiver Wirkung.

5. Verwendung von trans-3,4-Diphenylpiperidin bei der Herstellung eines Arzneimittels mit antidepressiver Wirkung.

6. Verfahren zur Herstellung von substituierten cis-und trans-3,4-Diphenylpiperidinderivaten der Formel I

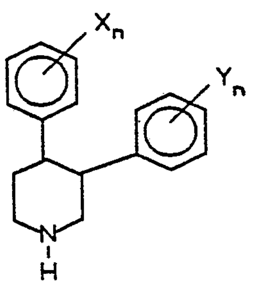

I

worin
X und Y gleich oder verschieden sind und für Halogen, $C_1$-$C_4$-Alkyl, $C_5$-$C_6$-Cycloalkyl, Phenyl-($C_1$-$C_3$)alkyl, Trifluormethyl, Trichlormethyl, Hydroxy, $C_1$-$C_4$-Alkoxy, Halogen-($C_1$-$C_4$)-alkoxy, Phenyloxy, Phenyl($C_1$-$C_3$)-alkoxy, Acyloxy mit bis zu 4 Kohlenstofffatomen, Nitro, N,N-Dialkylamino mit jeweils 1 bis 4 Kohlenstoffatomen in den Alkylresten, Morpholino oder $C_1$-$C_4$-Alkylsulfonyl stehen oder einer der Reste X oder Y Wasserstoff darstellt, und
n eins, zwei oder drei bedeutet,
oder eines physiologisch verträgliches Salz dieser Verbindungen, ausgenommen Verbindungen, worin einer der Substituenten X oder Y $C_1$-$C_4$-Alkoxy und der andere Wasserstoff und n gleich 2 ist,
dadurch gekennzeichnet, daß man ein Reduktionsmittel für die Amidfunktion eines Piperidons auf ein Piperidon der Formel II

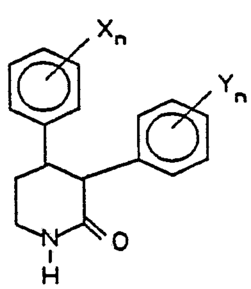

II

worin X, Y und n die zu Formel I angegebenen Bedeutungen haben, einwirken läßt, um das entsprechende Piperidin I derselben cis-oder trans-Stereochemie wie des Ausgangspiperidons zu erhalten, und das Reaktionsprodukt gegebenenfalls in ein physiologisch verträgliches Salz überführt.